# EUROPÄISCHE PATENTSCHRIFT

(11) **EP 0 904 111 B1**
(45) Veröffentlichungstag und Bekanntmachung des Hinweises auf die Patenterteilung: **30.07.2003**
(21) Anmeldenummer: 97925952.0
(22) Anmeldetag: 27.05.1997
(51) Int. Cl.: A61K 47/08, A61K 7/48

(54) **KOSMETISCHE UND/ODER PHARMAZEUTISCHE EMULSIONEN ENTHALTEND BEHENYLDERIVATE**
COSMETIC AND/OR PHARMACEUTICAL EMULSIONS CONTAINING BEHENYL DERIVATIVES
EMULSIONS COSMETIQUES ET/OU PHARMACEUTIQUES CONTENANT DES DERIVES DE BEHENYLE

(30) Priorität: 05.06.1996 DE 19622613
(43) Veröffentlichungstag der Anmeldung: 31.03.1999
(73) Patentinhaber: Cognis Deutschland GmbH & Co. KG, 40589 Düsseldorf (DE)
(72) Erfinder: LE HEN FERRENBACH, Catherine, F-77100 Meaux (FR)
(86) Internationale Anmeldenummer: EP9702735
(87) Internationale Veröffentlichungsnummer: WO97046258

(56) Entgegenhaltungen:
- DE-A- 3 313 002
- US-A- 4 597 963
- PATENT ABSTRACTS OF JAPAN vol. 010, no. 203 (C-360), 16.Juli 1986 & JP 61 047408 A (POLA CHEM IND INC), 7.März 1986,
- PATENT ABSTRACTS OF JAPAN vol. 011, no. 192 (C-429), 19.Juni 1987 & JP 62 012708 A (NIKKO KEMIKARUZU KK), 21.Januar 1987,

## Beschreibung

### Gebiet der Erfindung

Die Erfindung betrifft kosmetische und pharmazeutische Emulsionen mit verbesserter Stabilität, enthaltend Behenverbindungen und Ölkörper sowie die Verwendung der Behenverbindungen als Emulgatoren zur Herstellung von kosmetischen und/oder pharmazeutischen Zubereitungen.

### Stand der Technik

Zur Herstellung kosmetischer und pharmazeutischer Mittel, wie beispielsweise Cremes, Lotionen oder Salben, werden neben Ölkörpern in vielen Fällen auch nichtionische Emulgatoren wie beispielsweise Anlagerungsprodukte von Ethylenoxid an Cetearylalkohol eingesetzt. Es zeigt sich dabei, daß die resultierenden Emulsionen zwar bei Raumtemperatur stabil sind und eine ausreichend hohe Viskosität aufweisen, die jedoch bei längerer Lagerung, zumal bei Temperaturbelastung, allmählich zusammenbricht. Es entstehen dünnflüssige Produkte, in einer Reihe von Fällen kann es auch zu Entmischungen kommen. Ein weiterer Nachteil besteht zudem darin, daß die nichtionischen Emulgatoren zwar in der Regel eine sehr gute dermatologische Verträglichkeit besitzen, bei Verbrauchern mit besonders empfindlicher Haut, zumal bei häufiger topischer Anwendung, dennoch aber Irritationen hervorrufen können.

Die JP-A-61047408 betrifft kosmetische Öl-in-Wasser-Emulsionen auf Basis spezieller alkoxylierter C₁₀-C₂₂-Alkohole als Emulgatoren, welche die Emulgierbarkeit und die Stabilität der Emulsion verbessern. Bezüglich der Stabilität bei längerer Lagerung unter Temperaturbelastung sind diese Produkte jedoch noch verbesserungswürdig.

Die Aufgabe der Erfindung hat somit darin bestanden, Emulsionen für kosmetische oder pharmazeutische Anwendungen auf Basis nichtionischer Tenside zur Verfügung zu stellen, die frei von den geschilderten Nachteilen des Stands der Technik sind, d.h. die auch bei Temperaturbelastung über längere Zeit lagerstabil sind und eine verbesserte dermatologische Verträglichkeit aufweisen.

### Beschreibung der Erfindung

Gegenstand der Erfindung sind kosmetische und/oder pharmazeutische Emulsionen, enthaltend
(a) Emulgatoren ausgewählt aus der Gruppe, die gebildet wird von Behenylethoxylaten, Behenylestem und/oder Behensäureestem der Formel **R**^{**4**}**CO-(OCH**_{**2**}**CH**_{**2**}**)**_{**p**}**OR**^{**5**}**, (III)**, in der R⁴CO für einen Behensäurerest, R⁵ für einen Alkylrest mit **1** bis **4** Kohlenstoffatomen und p für 0 oder Zahlen von 1 bis 30 steht, und
(b) Ölkörper.

Überraschenderweise wurde gefunden, daß die genannten Mischungen von Behenverbindungen und Ölkörpem zusammen mit wäßrigen Phasen Emulsionen ergeben, die besonders lagerstabil sind, d.h. deren Viskosität sich auch bei längerer Lagerung bei erhöhten Temperaturen nicht verändert. Die Emulsionen zeichnen sich zudem durch eine besonders hohe dermatologische Verträglichkeit aus.

### Behenylethoxylate

Unter Behenylethoxylaten, die als Emulgatoren in Betracht kommen, sind Anlagerungsprodukte von Ethylenoxid an technische Behenylalkohole zu verstehen, die der Formel (I) folgen,

**R**^{**1**}**O(CH**_{**2**}**CH**_{**2**}**O)**_{**n**}**H** (I)

in der R¹ für einen Behenylrest und n für Zahlen von 1 bis 30 steht. Die Behenylalkohole können dabei in untergeordneten Mengen, d.h. in Mengen unterhalb von 20, vorzugsweise unterhalb von 10 und insbesondere unterhalb von 5 Gew.-% andere Fettalkohole wie beispielsweise Cetylalkohol, Stearylalkohol, Oleylalkohol und/oder Erucaalkohol enthalten. Die Ethoxylate sind in an sich bekannter Weise herstellbar, d.h. durch Ethoxylierung der Ausgangsalkohole in Gegenwart homogener oder heterogener basischer Katalysatoren wie beispielsweise Natriummethylat oder calciniertem Hydrotalcit. Dementsprechend können die Ethoxylate eine konventionell breite oder aber eine eingeengte Homologenverteilung aufweisen. Besonders bevorzugt ist der Einsatz von Anlagerungsprodukten von 10 bis 20 Mol Ethylenoxid an Behenylalkohol.

### Behenylester

Als weitere geeignete Behenverbindungen kommen Behenylester in Betracht, bei denen es sich um gegebenenfalls ethoxylierte Ester von Carbonsäuren mit technischem Behenylalkohol handelt, die der Formel **(II)** folgen,

**R**^{**2**}**CO-(OCH**_{**2**}**CH**_{**2**}**)**_{**m**}**OR**^{**3**} (II)

in der R²CO für einen Acylrest mit 1 bis 22 Kohlenstoffatomen, R³ für einen Behenylrest und m für 0 oder Zahlen von 1 bis 30 steht. Typische Beispiele sind Ester der Essigsäure, Propionsäure, Buttersäure, Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen mit technischem Behenylalkohol. Wie schon ausgeführt, ist hierunter eine technische Qualität des Behenylalkohols zu verstehen, die herstellungsbedingt noch in untergeordneten Mengen andere langkettige Fettalkohole enthalten kann. Des weiteren können die Behenylester 1 bis 30, vorzugsweise 10 bis 20 Mol Ethylenoxid enthalten. Die Herstellung dieser Ester kann sowohl durch Veresterung der Carbonsäuren mit den ethoxylierten Behenylalkoholen als auch durch nachträgliche Ethoxylierung der Ester beispielsweise in Gegenwart von calciniertem Hydrotalcit als Ethoxylierungskatalysator erfolgen. Als Emulgatoren bevorzugt sind indes Ester von Carbonsäuren mit 1 bis 6 Kohlenstoffen mit Behenylalkohol bzw. Addukten von durchschnittlich 10 bis 20 Mol Ethylenoxid an Behenylalkohol.

### Behensäureester

Bei den weiterhin als Emulgatorkomponente in Betracht kommenden Behensäureestem handelt es sich um gegebenenfalls ethoxylierte Ester der Behensäure mit niederen aliphatischen Alkoholen, die der Formel **(III)** folgen,

**R**^{**4**}**CO-(OCH**_{**2**}**CH**_{**2**}**)**_{**p**}**OR**^{**5**} (III)

in der R⁴CO für einen Behensäurerest, R⁵ für einen Alkylrest mit 1 bis 4 Kohlenstoffatomen und p für 0 oder Zahlen von 1 bis 30 steht. Typische Beispiele sind die Methyl-, Ethyl-, n-Propyl-, Isopropyl- und Buttersäureester der Behensäure, wobei Behensäuremethylester als Emulgator besonders bevorzugt ist. Des weiteren können die Behensäureester 1 bis 30, vorzugsweise 10 bis 20 Mol Ethylenoxid enthalten. Die Herstellung dieser Ester kann durch Veresterung der Behensäure mit den ethoxylierten Alkoholen, vorzugsweise jedoch durch nachträgliche Ethoxylierung der Ester beispielsweise in Gegenwart von calciniertem Hydrotalcit als Ethoxylierungskatalysator erfolgen. Bevorzugt ist der Einsatz von Umsetzungsprodukten von 10 bis 20 Mol Ethylenoxid mit Behensäuremethylester.

Die erfindungsgemäßen Emulsionen können die Emulgatoren in Mengen von 1 bis 15 und vorzugsweise 5 bis 10 Gew.-% - bezogen auf die Emulsionen - enthalten.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. naphthenische Kohlenwasserstoffe in Betracht. Die erfindungsgemäßen Emulsionen können die Ölkörper in Mengen von 10 bis 95 und vorzugsweise 25 bis 75 Gew.-% - bezogen auf die Emulsionen - enthalten.

Der Anteil der Ölkörper am nichtwäßrigen Anteil der Emulsionen kann 5 bis 99 und vorzugsweise 10 bis 75 Gew.-% ausmachen.

### Gewerbliche Anwendbarkeit

Die erfindungsgemäßen Emulsionen zeichnen sich auch bei Temperaturbelastung durch eine hohe Lagerstabilität sowie besonders vorteilhafte sensorische Eigenschaften und dermatologische Verträglichkeit aus. Ein weiterer Gegenstand der Erfindung betrifft daher die Verwendung der genannten Behenverbindungen als Emulgatoren zur Herstellung von kosmetischen und/oder pharmazeutischen Zubereitungen.

Der nichtwäßrige Anteil der Emulsionen, der sich weitgehend aus dem Emulgator- und dem Ölkörpergehalt zusammensetzt und dabei in der Regel dem Feststoffgehalt entspricht, liegt üblicherweise bei 5 bis 95 und vorzugsweise 15 bis 75 Gew.-%. Das bedeutet umgekehrt, daß die Emulsionen 5 bis 95 und vorzugsweise 25 bis 85 Gew.-% Wasser enthalten können, abhängig davon, ob Lotionen mit einer vergleichsweise niedrigen oder Cremes und Salben mit einer hohen Viskosität hergestellt werden sollen.

### Hilfs- und Zusatzstoffe

Die Emulsionen können als Hautpflegemittel, wie beispielsweise Tagescremes, Nachtcremes, Pflegecremes, Nährcreme, Bodylotions, Salben und dergleichen eingesetzt werden und als weitere Hilfs- und Zusatzstoffe Tenside, Co-Emulgatoren, Überfettungsmittel, Konsistenzgeber, Verdickungsmittel, Fette, Wachse, Stabilisatoren, biogene Wirkstoffe, Glycerin, Konservierungsmittel, Farb- und Duftstoffe enthalten.

Typische Beispiele für geeignete **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Ethercarbonsäuren, Alkyloligoglucoside, Fettsäureglucamide, Alkylamidobetaine und/oder vorzugsweise pflanzliche Proteinfettsäurekondensate.

Als **Co-Emulgatoren** können nichtionogene, ampholytische und/oder zwitterionische grenzflächenaktive Verbindungen verwendet werden, die sich durch eine lipophile, bevorzugt lineare Alkyl- oder Alkenylgruppe und mindestens eine hydrophile Gruppe auszeichnen. Diese hydrophile Gruppe kann sowohl eine ionogene als auch eine nichtionogene Gruppe sein. Nichtionogene Emulgatoren enthalten als hydrophile Gruppe z. B. eine Polyolgruppe, eine Polyalkylenglycolethergruppe oder eine Kombination aus Polyol- und Polyglycolethergruppe.

Bevorzugt sind solche Mittel, die als **O/W-Co-Emulgatoren** nichtionogene Tenside aus mindestens einer der folgenden Gruppen enthalten:
(a1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(a2) C_{12/18}-Fettsäuremono- und -diester von Anlagerungsprodukten von 1 bis 30 Mol Ethylenoxid an Glycerin;
(a3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(a4) Alkylmono- und -oligoglycoside mit 8 bis 22 Kohlenstoffatomen im Alkylrest und deren ethoxylierte Analoga;
(a5) Anlagerungsprodukte von 15 bis 60 Mol Ethylenoxid an Ricinusöl und/oder gehärtetes Ricinusöl;
(a6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Als **W/O-Co-Emulgatoren** kommen in Betracht:
(b1) Anlagerungsprodukte von 2 bis 15 Mol Ethylenoxid an Ricinusöl und/oder gehärtetes Ricinusöl;
(b2) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b3) Trialkylphosphate;
(b4) Wollwachsalkohole;
(b5) Polysiloxan-Polyalkyl-Polyether-Copolymere bzw. entsprechende Derivate;
(b6) Mischester aus Pentaerythrit, Fettsäuren, Citronensäure und Fettalkohol gemäß **DE-PS 11 65 574** sowie
(b7) Polyalkylenglycole.

Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen, Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdikkungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Pflanzenextrakte und Vitaminkomplexe zu verstehen. Als **Konservierungsmittel** eignen sich beispielsweise Phenoxyethanol, Formaldehydlösung, Parabene, Pentandiol oder Sorbinsäure. Als **Periglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt. Der Gesamtanteil der Hilfs- und Zusatzstoffe kann 1 bis 50, vorzugsweise 5 bis 40 Gew.-% - bezogen auf die Mittel - betragen.

Typische Emulsionen weisen Zusammensetzungen gemäß Tabelle 1 auf (mit der Maßgabe, daß sich die Angaben zu 100 Gew.-% ergänzen).

**Tabelle 1**

| **Typische Zusammensetzung von Lotionen und Cremes** | | |
|---|---|---|
| **Bestandteil** | **Lotion [Gew.-%]** | **Creme [Gew.-%]** |
| Behenylethoxylate | 1 - 5 | 1 - 10 |
| Ölkörper | 25 - 50 | 25 - 50 |
| Hilfs- und Zusatzstoffe | 1 - 5 | 1 - 5 |
| Wasser | 50 - 70 | 25 bis 50 |

Der Gesamtanteil der Hilfs- und Zusatzstoffe kann 1 bis 10, vorzugsweise 2 bis 5 Gew.-% - bezogen auf die Mittel - betragen. Die Herstellung der Mittel kann in an sich bekannter Weise, d.h. beispielsweise durch Heiß-, Heiß-Heiß/Kalt- bzw. PIT-Emulgierung erfolgen. Hierbei handelt es sich um ein rein mechanisches Verfahren, eine chemische Reaktion findet nicht statt.

### Beispiele

Es wurden Emulsionen hergestellt, enthaltend 5 Gew.-% Emulgatoren (A1 bis A4), 35 Gew.-% Ölkörper (B1 bis B3) und ad 100 Gew.-% Wasser. Die Herstellung der Emulsionen erfolgte nach der PIT-Methode, also oberhalb der Phaseninversionstemperatur. Die Rezepturen R1 bis R4 sind erfindungsgemäß, die Rezepturen R5 und R6 dienen zum Vergleich. Die Viskosität der Proben wurde nach der Brookfield-Methode in einem RVF-Viskosimeter (20 Upm, Spindel 1) sofort sowie nach Lagerung über 7 Tage bei 20 bzw. 40°C bestimmt. Die Beurteilung der dermatologischen Verträglichkeit erfolgte an der Chorionallantoismembran des bebrüteten Hühnereis (HET-CAM), die ein Modell für die Augenschleimhautverträglichkeit einer Substanz darstellt. Bestimmt wurde die Cytotoxizität relativ zur Vergleichsrezeptur R5. Die Ergebnisse sind in Tabelle 2 zusammengefaßt.

**Tabelle 2**

| **Viskosität und Lagerstabilität (Mengenangaben als Gew.-%)** | | | | | | |
|---|---|---|---|---|---|---|
| **Komponenten** | **R1 %** | **R2 %** | **R3 %** | **R4 %** | **R5 %** | **R6 %** |
| Behenylalkohol+10 EO | 5 | - | - | - | - | - |
| Behenylalkohol+20 EO | - | 5 | | | - | - |
| Essigsäurebehenylester+10 EO | - | - | 5 | | - - | - |
| Behensäuremethylester+10 EO | - | - | - | 5 | - | - |
| Cetearylalkohol+10 EO | - | - | - | - | 5 | - |
| Cetearylalkohol+20 EO | - | - | - | - | - | 5 |
| Dicaprylyl Ether | 15 | 15 | 15 | 15 | 15 | 15 |
| Decyl Oleate | 10 | 10 | 10 | 10 | 10 | 10 |
| Almond Oil | 5 | 5 | 5 | 5 | 5 | 5 |
| Wasser | ad 100 | | | | | |
| ***Cytotoxizität [%-rel]*** | 84 | 80 | 89 | 91 | 100 | 97 |
| ***Viskosität [mPas]*** | | | | | | |
| ***- sofort*** | 8.000 | 10.000 | 10.000 | 10.000 | 8.000 | 10.000 |
| ***- nach 7 d, 20°C*** | 7.800 | 9.800 | 9.800 | 9.500 | 6.000 | 8.500 |
| ***- nach 7 d, 40°C*** | 7.800 | 9.700 | 9.500 | 9.400 | 4.500 | 6.000 |

Man erkennt, daß lagerstabile Emulsionen mit minimierter Cytotoxizität nur unter Verwendung der erfindungsgemäßen Behenylverbindungen erhalten werden.

## Patentansprüche

1. Kosmetische und/oder pharmazeutische Emulsionen, enthaltend
(a) Emulgatoren ausgewählt aus der Gruppe, die gebildet wird von Behenylethoxylaten, Behenylestem und/oder Behensäureestem der Formel **R**^{**4**}**CO-(OCH**_{**2**}**CH**_{**2**}**)**_{**p**}**OR**^{**5**}**, (III)**, in der R⁴CO für einen Behensäurerest, R⁵ für einen Alkylrest mit 1 bis 4 Kohlenstoffatomen und p für 0 oder Zahlen von 1 bis 30 steht, und
(b) Ölkörper.

2. Emulsionen nach Anspruch 1, **dadurch gekennzeichnet, daß** sie als Emulgatoren Behenylethoxylate der Formel (I) enthalten,
**R**^{**1**}**O(CH**_{**2**}**CH**_{**2**}**O)**_{**n**}**H** (I)
in der R¹ für einen Behenylrest und n für Zahlen von 1 bis 30 steht.

3. Emulsionen nach den Ansprüchen 1 und 2, **dadurch gekennzeichnet, daß** sie als Emulgatoren Behenylester der Formel **(II)** enthalten,
**R**^{**2**}**CO-(OCH**_{**2**}**CH**_{**2**}**)**_{**m**}**OR**^{**3**} (II)
in der R²CO für einen Acylrest mit 1 bis 22 Kohlenstoffatomen, R³ für einen Behenylrest und m für 0 oder Zahlen von 1 bis 30 steht.

4. Emulsionen nach den Ansprüchen 1 bis 3, **dadurch gekennzeichnet, daß** sie Ölkörper enthalten, die ausgewählt sind aus der Gruppe, die gebildet wird von Guerbetalkoholen auf Basis von Fettalkoholen mit 6 bis 18 Kohlenstoffatomen, Estem von linearen C₆-C₂₀-Fettsäuren mit linearen C₆-C₂₀-Fettalkoholen, Estem von verzweigten C₆-C₁₃-Carbonsäuren mit linearen C₆-C₂₀-Fettalkoholen, Estem von linearen C₆-C₁₈-Fettsäuren mit verzweigten Alkoholen, Estem von linearen und/oder verzweigten Fettsäuren mit mehrwertigen Alkoholen und/oder Guerbetalkoholen, Triglyceriden auf Basis C₆-C₁₀-Fettsäuren, pflanzlichen Ölen, verzweigten primären Alkoholen, substituierten Cyclohexanen, Guerbetcarbonaten, Dialkylethern und/oder aliphatischen bzw. naphthenischen Kohlenwasserstoffen.

5. Emulsionen nach den Ansprüchen 1 bis 4, **dadurch gekennzeichnet, daß** sie die Emulgatoren in Mengen von 1 bis 15 Gew.-%- bezogen auf die Emulsionen - enthalten.

6. Emulsionen nach den Ansprüchen 1 bis 5, **dadurch gekennzeichnet, daß** sie die Ölkörper in Mengen von 10 bis 95 Gew.-% - bezogen auf die Emulsionen - enthalten.

7. Emulsionen nach den Ansprüchen 1 bis 6, **dadurch gekennzeichnet, daß** sie einen nicht-wäßrigen Anteil von 5 bis 95 Gew.-% aufweisen.

8. Verwendung von Behenverbindungen nach Anspruch 1 als Emulgatoren zur Herstellung von kosmetischen und/oder pharmazeutischen Zubereitungen.

## Claims

1. Cosmetic and/or pharmaceutical emulsions containing
(a) emulsifiers selected from the group consisting of behenyl ethoxylates, behenyl esters and/or behenic acid esters corresponding to the formula **R**^{**4**}**CO-(OCH**_{**2**}**CH**_{**2**}**)**_{**p**}**OR**^{**5**} **(III),** where R⁴CO is a behenic acid residue, R⁵ is a C₁₋₄ alkyl group and p = 0 or a number of 1 to 30, and
(b) oil components.

2. Emulsions as claimed in claim 1, **characterized in that** they contain behenyl ethoxylates corresponding to formula (I):
**R**^{**1**}**O(CH**_{**2**}**CH**_{**2**}**O)**_{**n**}**H** (I)
in which R¹ is a behenyl group and n is a number of 1 to 30, as emulsifiers.

3. Emulsions as claimed in claims 1 and 2, **characterized in that** they contain behenyl esters corresponding to formula (II):
**R**^{**2**}**CO-(OCH**_{**2**}**CH**_{**2**}**)**_{**m**}**OR**^{**3**} (II)
in which R²CO is an acyl group containing 1 to 22 carbon atoms, R³ is a behenyl group and m is 0 or a number of 1 to 30,
as emulsifiers.

4. Emulsions as claimed in claims 1 to 3, **characterized in that** they contain oil components selected from the group consisting of Guerbet alcohols based on C₆₋₁₈ fatty alcohols, esters of linear C₆₋₂₀ fatty acids with linear C₆₋₂₀ fatty alcohols, esters of branched C₆₋₁₃ carboxylic acids with linear C₆₋₂₀ fatty alcohols, esters of linear C₆₋₁₈ fatty acids with branched alcohols, esters of linear and/or branched fatty acids with polyhydric alcohols and/or Guerbet alcohols, triglycerides based on C₆₋₁₀ fatty acids, vegetable oils, branched primary alcohols, substituted cyclohexanes, Guerbet carbonates, dialkyl ethers and/or aliphatic or naphthenic hydrocarbons.

5. Emulsions as claimed in claims 1 to 4, **characterized in that** they contain the emulsifiers in quantities of 1 to 15% by weight, based on the emulsions.

6. Emulsions as claimed in claims 1 to 5, **characterized in that** they contain the oil components in quantities of 10 to 95% by weight, based on the emulsions.

7. Emulsions as claimed in claims 1 to 6, **characterized in that** they have a nonaqueous component of 5 to 95% by weight.

8. The use of the behenyl compounds according to claim 1 as emulsifiers for the production of cosmetic and/or pharmaceutical preparations.

## Revendications

1. Emulsions cosmétiques et/ou pharmaceutiques contenant :
a) des agents émulsionnants choisis dans le groupe formé d'éthoxylates de béhényle, d'esters de béhényle et/ou d'esters d'acide béhénique de formule
**R**^{**4**}**CO-(OCH**_{**2**}**CH**_{**2**}**)**_{**P**}**OR**^{**5**} (III),
dans laquelle
R⁴CO représente un reste d'acide béhénique,
R⁵ représente un reste alkyle ayant de 1 à 4 atomes de carbone, et
p représente 0 ou des nombres allant de 1 à 30, et
b) des composés huileux.

2. Emulsions selon la revendication 1,
**caractérisées en ce qu'**
elles renferment comme agents émulsionnants des éthoxylates de bénényle de formule (I)
**R**^{**1**}**O(CH**_{**2**}**CH**_{**2**}**O)**_{**n**}**H** (I)
dans laquelle
R¹ représente un reste béhényle et un des nombres allant de 1 à 30.

3. Emulsions selon les revendications 1 et 2,
**caractérisées en ce qu'**
elles renferment comme agents émulsionnants des esters de béhényle de formule (II)
**R**^{**2**}**CO-(OCH**_{**2**}**CH**_{**2**}**)**_{**m**}**OR**^{**3**} (II)
dans laquelle
R²CO représente un reste acyle ayant de 1 à 22 atomes de carbone,
R³ représente un reste béhényle et
m représente 0 ou des nombres allant de 1 à 30.

4. Emulsions selon les revendications 1 à 3,
**caractérisées en ce qu'**
elles renferment des composés huileux qui sont choisis dans le groupe formé d'alcools de Guerbet à base d'alcools gras ayant de 6 à 18 atomes de carbone, d'esters d'acides gras linéaires en C₆-C₂₀, avec des alcools gras linéaires en C₆-C₂₀, des esters d'acides gras ramifiés en C₆-C₁₃ avec des alcools gras linéaires en C₆-C₂₀, des esters d'acides gras linéaires en C₆-C₁₈ avec des alcools ramifiés, des esters d'acides gras linéaires et/ou ramifiés avec des alcools plurifonctionnels et/ou des alcools de Guerbet, des triglycérides à base d'acides gras enC₆-C₁₀, des huiles végétales, des alcools primaires ramifiés, des cyclohexanes substitués, des carbonates de Guerbet, des éthers dialkyliques, et/ou des hydrocarbures aliphatiques en naphténiques.

5. Emulsions selon les revendications 1 à 4,
**caractérisées en ce qu'**
elles renferment les agents émulsionnants en des quantités allant de 1 à 15 % en poids rapporté aux émulsions.

6. Emulsions selon les revendications 1 à 5,
**caractérisées en ce qu'**
elles renferment les composés huileux en des quantités allant de 10 à 95 % en poids rapporté aux émulsions.

7. Emulsions selon les revendications 1 à 6,
**caractérisées en ce qu'**
elles possèdent une proportion non aqueuse de 5 à 95 % en poids.

8. Utilisation de composés béhéniques selon la revendication 1,
en tant qu'agents émulsionnants en vue de la production de préparations cosmétiques et/ou pharmaceutiques.
